(19) Europäisches Patentamt
European Patent Office
Office européen des brevets

(11) EP 1 448 668 B1

(12) EUROPEAN PATENT SPECIFICATION

(45) Date of publication and mention of the grant of the patent:
**09.08.2006 Bulletin 2006/32**

(21) Application number: **02789715.6**

(22) Date of filing: **18.11.2002**

(51) Int Cl.:
***C08G 65/48*** *(2006.01)* ***C08G 63/692*** *(2006.01)*
***B01J 31/18*** *(2006.01)* ***B01J 31/16*** *(2006.01)*

(86) International application number:
**PCT/US2002/036919**

(87) International publication number:
**WO 2003/046049 (05.06.2003 Gazette 2003/23)**

# (54) POLYMERIC, PHOSPHOROUS-CONTAINING COMPOSITIONS AND THEIR USE IN HYDROCYANATION, ISOMERIZATION AND HYDROFORMYLATION REACTIONS

POLYMERE PHOSPHORHALTIGE ZUSAMMENSETZUNGEN UND DEREN VERWENDUNG IN HYDROCYANIERUNGS-, ISOMERISIERUNGS- UND HYDROFORMYLIERUNGS-REAKTIONEN

COMPOSITIONS POLYMERES CONTENANT DU PHOSPHORE ET LEUR UTILISATION EN REACTION D'HYDROCYANATION, D'ISOMERISATION ET D'HYDROFORMYLATION

(84) Designated Contracting States:
**DE ES FR GB IT NL**

(30) Priority: **26.11.2001 US 994097**

(43) Date of publication of application:
**25.08.2004 Bulletin 2004/35**

(60) Divisional application:
**05076031.3 / 1 571 172**
**05076346.5 / 1 586 598**

(73) Proprietor: **E. I. du Pont de Nemours and Company**
**Wilmington,**
**Delaware 19898 (US)**

(72) Inventors:
- **RADU, Nora, S.**
  **Landenberg, PA 19350 (US)**
- **TAM, Wilson**
  **Boothwyn, PA 19061 (US)**

(74) Representative: **Cockerton, Bruce Roger et al**
**Carpmaels & Ransford,**
**43 Bloomsbury Square**
**London WC1A 2RA (GB)**

(56) References cited:
**WO-A-01/21308** **WO-A-01/21684**
**US-A- 6 121 184**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 448 668 B1

## Description

## BACKGROUND OF THE INVENTION

### FIELD OF THE INVENTION

The present invention relates to the preparation of polymeric, phosphorus-containing ligand compositions. The present invention also relates to catalyst compositions involving a Group VIII metal in the presence of the polymeric ligands and use of such catalysts in hydrocyanation, isomerization, and hydroformylation reactions.

### DESCRIPTION OF THE RELATED ART

Phosphorus-based ligands are generally known in catalysis, finding use for a number of commercially important chemical transformations. Phosphorus-based ligands commonly encountered in catalysis include phosphines, phosphinites, phosphonites and phosphites. Monodentate phosphorous ligands, e.g. monophosphine and monophosphite ligands, are compounds that usually contain a single phosphorus atom that serves as an electron donor to a transition metal. Bidentate phosphorous ligands, e.g. bisphosphine, bisphosphinite, bisphosphonite, bisphosphite, and bis(phosphorus) ligands, in general, contain two phosphorus electron donor atoms and typically form cyclic chelate structures with transition metals.

Two catalytic reactions of particular industrial importance using phosphorous ligands are olefin hydrocyanation and isomerization of branched nitriles to linear nitriles. Phosphite and phosphinite ligands are particularly good ligands for both reactions. The hydrocyanation of ethylenically unsaturated compounds (olefins) using transition metal complexes with monodentate phosphite ligands is well documented in the prior art. See for example U. S. Pat. Nos. 3,496,215; 3,631,191; 3,655,723; 3,766,237; and 5,543,536. Bidentate phosphite ligands have also been shown to be particularly useful ligands in the hydrocyanation of activated ethylenically unsaturated compounds. See for example, J. Chem. Soc., Chem. Commun., 1991, 1292; J.Chem. Soc., Chem. Commun., 1991, 803; PCT Pat. App. WO 9303839; and U. S. Pat. Nos. 5,512,696; 5,723,641; and 5,688,986. Bidentate phosphinite and phosphonite ligands are described in U.S. Pat. Nos. 5,817,850; 5,523,453; and 5,693,843, and PCT Pat. Apps. WO 9964155, WO 9913983, WO 9946044, and WO 9843935.

Hydroformylation is another industrially useful process that utilizes catalysts made from phosphorus-containing ligands. The use of phosphine ligands, including diphosphines, is known for this purpose. The use of catalysts made from phosphite ligands is also known. Such catalysts usually contain a Group VIII metal. See for example, U. S. Pat. No. 5,235,113.

Recovery of the ligand and catalyst is important for a successful commercial process. Typical separation procedures to remove the product(s) from the catalyst and ligand involve extraction with an immiscible solvent or distillation. It is usually difficult to recover the catalyst and ligand quantitatively. For instance, distillation of a volatile product from a non-volatile catalyst results in thermal degradation of the catalyst. Similarly, extraction results in some loss of catalyst into the product phase. For extraction, one would like to be able to select and/or control the solubility of the ligand and catalyst to disfavor solubility in the product phase. These ligands and metals are often very expensive and thus it is important to keep such losses to a minimum for a commercially viable process.

One method to solve the problem of catalyst and product separation is to attach the catalyst to an insoluble support. Examples of this approach have been previously described, and general references on this subject can be found in "Supported Metal Complexes", D. Reidel Publishing, 1985; Acta Polymer., 1996, 47, 1; "Comprehensive Organometallic Chemistry", Pergamon Press, 1982, Chapter 55; J. Mol. Catal. A, 1995, 104, 17 and Macromol. Symp., 1994, 80, 241. Specifically, monophosphine and monophosphite ligands attached to solid supports are described in these references. Bisphosphine ligands have also been attached to solid supports and used for catalysis, as described in, for example, U. S. Pat. Nos. 5,432,289 and 5,990,318; J. Mol. Catal. A, 1996, 112, 217; J.Chem. Soc., Chem. Commun., 1996, 653; J. Org. Chem., 1998, 63, 3137; Spec. Chem., 1998, 18, 224 and PCT Pat. App. WO 9812202. PCT Pat. Apps. WO 9906146 and WO 9962855 show the use of supported phosphorus ligands in hydrocyanation and hydroformylation reactions, respectively. Bisphosphite ligands have also been grafted to solid supports such as those described in U. S. Pat. No. 6,121,184. The solid support in these prior art examples can be organic, e.g., a polymer resin, or inorganic in nature.

Polymer-supported multidentate phosphorous ligands may be prepared by a variety of methods known in the art, as described in U. S. Pat. Nos. 4,769,498 and 4,668,651, PCT Pat. Apps. WO 9303839 and WO 9906146, and European Pat.Apps. EP 0864577 A2 and EP 0877029 A2. The prior art discloses side-chain polymers containing multidentate phosphorous ligands as pendant groups.

Another method to solve the problem of separating the catalyst from the reaction product is to copolymerize phosphorus-containing ligands with other non-ligand monomers to produce insoluble phosphorus-containing ligands.

Examples of such polymer-immobilized phosphine ligands have been reported in J. Am. Chem. Soc., 2000, 122, 6217 and J. Org. Chem., 1986, 51, 4189. In addition, polymer-immobilized phosphine-phosphite ligands and their use in hydroformylation catalysis recently have been described in Bull. Chem. Soc. Jpn., 1999, 72, 1911; J. Am. Chem. Soc., 1998, 120, 4051; and European Pat. App. EP 0864577.

## BRIEF SUMMARY OF THE INVENTION

The present invention provides a method to produce a polymeric phosphorus-containing composition by:

1. aryl to aryl oxidative coupling of a composition comprising a monomer having the structure:

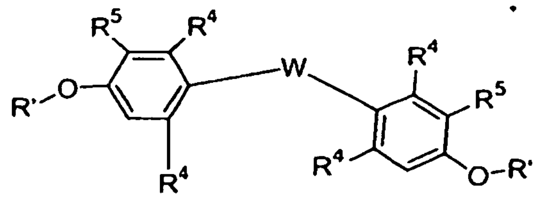

wherein:

W is $C_6$-$C_{20}$ arylene, $C_1$-$C_{20}$ alkylene or $C_1$-$C_{20}$ cycloalkylene;
each R' is individually hydrogen or a hydroxyl protective group selected from, but not limited to, alkyl, alkoxyalkyl, carbonylalkyl, or a crown ether formed by taking both R' groups together;
each $R^4$ is independently H, $C_1$ to $C_{20}$ branched or straight chain alkyl, $C_1$ to $C_{20}$ cycloalkyl, or $C_6$ to $C_{20}$ aryl; and,
each $R^5$ is independently $C_1$ to $C_{20}$ branched or straight chain alkyl, $C_1$ to $C_{20}$ cycloalkyl, or $C_6$ to $C_{20}$ aryl and,

2. if R' is a hydroxyl protective group, converting R' to H or alkali metal or alkaline earth metal, and
3. phosphonylating the product of step (1) if R' is other than a hydroxyl protective group, or phosphonylating the product of steps (1) and (2) if R' is a hydroxyl protective group, with at least one diaryloxyphosphite [-P(-O-Ar)$_2$], diarylphosphine [-R(Ar)$_2$], and/or aryl,aryloxyphosphinite [-P(Ar)(-O-Ar)], wherein each Ar is individually phenyl, substituted phenyl, naphthyl, or substituted naphthyl, provided that the two Ar groups that are directly or indirectly bonded to the same phosphorus atom may be linked to each other by a linking unit selected from direct bond, alkylidene, secondary or tertiary amine, oxygen, sulfide, sulfone, and sulfoxide.

Another aspect of the present invention provides a polymeric, phosphorus-containing composition made as described above.

A further aspect of the present invention provides a catalyst comprising at least one of the polymeric, phosphorus-containing compositions made as described above and at least one Group VIII metal.

A further aspect of the present invention provides the use of the above catalyst composition for a hydrocyanation process comprising reacting an unsaturated organic compound with HCN in the presence of the polymeric, phosphorus-containing composition and the Group VIII metal with or without a Lewis acid.

A further aspect of the present invention provides the use of the above catalyst composition for an isomerization process comprising reacting an unsaturated organic nitrile compound in the presence of the polymeric, phosphorus-containing bidentate ligand composition and a Group VIII metal.

A further aspect of the present invention provides the use of the above catalyst composition for a hydroformylation process comprising reacting an unsaturated organic compound with CO and $H_2$ in the presence of the polymeric, phosphorus-containing bidentate ligand composition and the Group VIII metal.

## DETAILED DESCRIPTION OF THE INVENTION

The phosphorus-containing compositions of the present invention may be viewed as belonging to the family of bidentate ligands, because each pair of trivalent phosphorus atoms is potentially available to simultaneously coordinately bond to a single Group VIII metal atom; i.e., the phosphorus atoms represent electron donors to the same metal atom of the resulting metal complex.

The pair of trivalent phosphorus atoms comprising the bidentate ligand moiety of the polymeric composition is

characterized as involving three phosphorus to oxygen bonds (i.e., a phosphite structure), one phosphorus to oxygen bond and two phosphorus to carbon bonds (i.e., a phosphinite structure), or two phosphorus to oxygen bonds and one phosphorus to carbon bond (i.e., a phosphonite structure). In each case, one of the phosphorus to oxygen bonds is associated with the oxygen of the hydroxyl group of the 2,2'-dihydroxyl-1,1'-binaphthalene or 2;2'-dihydroxyl-1,1'-biphenylene structures. The two other bonds associated with the trivalent phosphorus involve a pair of phosphorus to aryl carbon bonds, a pair of phosphorus to aryloxy oxygen bonds, or one phosphorus to aryl carbon bond and one phosphorus to aryloxy oxygen bond. Each aryl (Ar) or aryloxy (-O-Ar) is independently phenyl, naphthyl, substituted phenyl, or substituted naphthyl. For any individual trivalent phosphorus, the pair of aryl or aryloxy may optionally be linked to each other either directly or through a linking unit.

The present invention provides a method to prepare a polymeric, phosphorus-containing composition by:

(1) aryl to aryl oxidative coupling of monomer having the structure:

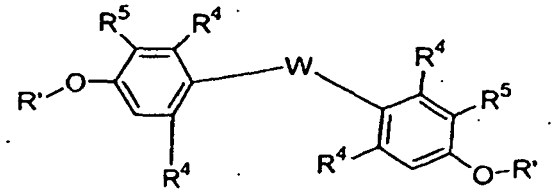

wherein:

W is $C_6$ to $C_{20}$ arylene, $C_1$ to $C_{20}$ alkylene or cycloalkylene;
each R' is individually hydrogen or a hydroxyl protective group selected from, but not limited to, alkyl, alkoxyalkyl, carbonylalkyl, or a crown ether formed by taking both R' groups together;
each $R^4$ is independently H, $C_1$ to $C_{20}$ branched or straight chain alkyl, $C_1$ to $C_{20}$ cycloalkyl, or $C_6$ to $C_{20}$ aryl; and
each $R^5$ is independently $C_1$ to $C_{20}$ branched or straight chain alkyl, $C_1$ to $C_{20}$ cycloalkyl, or $C_6$ to $C_{20}$ aryl; and

(2) phosphonylating with a substituent consisting of at least one diaryloxyphosphite [-P(-O-Ar)$_2$], diarylphosphine [-P(Ar)$_2$], and/or aryl,aryloxyphosphinite [-P(Ar)(-O-Ar)], where each Ar is individually phenyl, substituted phenyl, naptithyl, or substituted naphthyl, provided that the two Ar groups that are directly or indirectly bonded to the same phosphorus atom may be linked to each other by a linking unit selected from direct bond, alkylidene, secondary or tertiary amine, oxygen, sulfide, sulfone, and sulfoxide.

The W of the starting material is $C_6$-$C_{20}$ arylene, $C_1$ to $C_{20}$ alkylene or cycloalkylene. Examples of the monomer include, but are not limited to, those shown below.

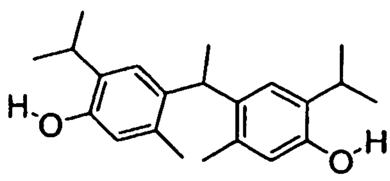

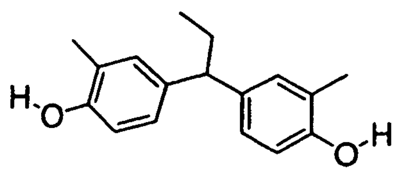

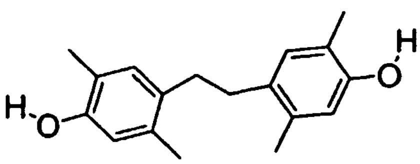

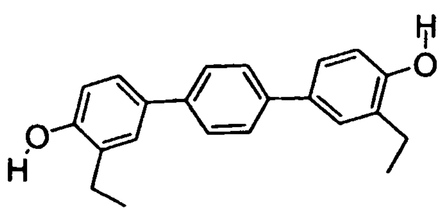

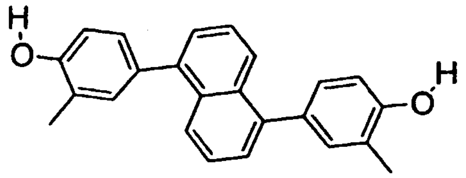
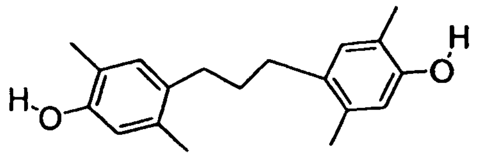

Oxidative coupling of phenols is a fundamental method for the synthesis of hydroxylated biaryls, as described in, for example: "Oxidative Coupling of Phenols", Ed.: W. I. Taylor, and A. R. Battersby, New York: M. Dekker Inc., 1967; Tetrahedron, 1992, 43, 9483; Organic Preparations and Procedures Int., 1975, 7, 255; J. Org. Chem., 1963, 28, 1063; J. Org. Chem., 1968, 34, 2388; J. Org. Chem., 1984, 49, 4456; J. Org. Chem., 1983, 48, 4948; J. Org. Chem., 1980, 45, 749; J. Org. Chem., 1981, 46, 4545; Tetrahedron Lett., 1977, 4447; Tetrahedron, 1992, 48, 9483; Photochemistry, 1988, 27, 3008. Coupling of compounds containing two phenol groups linked by a bridging group can lead to polymeric hydroxylated biaryls. An example is oxidative coupling of 4,4'-ethylidenebis(2-isopropyl-5-methylphenol). The starting material, 4,4'-ethylidenebis(2-isopropyl-5-methylphenol), was prepared using a procedure described in Bull. Chem. Soc. Jpn., 1989, 62, 3603, with the W group being $-C(CH_3)(H)-$. The pair of hydroxyl groups associated with the resultant 2,2'-dihydroxyl-1,1'-biphenylene structure is phosphonylated to produce a polymeric, phosphorus-containing, bidentate ligand composition.

The above processes describe the synthesis of polymeric, phosphorus-containing ligands of various structures. It is known in the art that the solubility properties of polymers are affected by their structure. It is preferred that the polymeric ligands of this invention be as insoluble as possible, consistent with retaining substantial catalytic activity. If the resulting polymers are insoluble, they can be separated by filtration from the reaction mixtures in which they are used and then recycled. If the polymeric ligands are partially soluble in the reaction mixture, they may be separated by filtration of the insoluble ligand and then precipitation of the soluble ligand with a solvent in which the ligand has extremely low solubility or by precipitation of the soluble ligand and filtration of the reaction mixture. If the ligands are completely soluble in the reaction mixture, they may be separated by precipitation with a solvent in which the ligand has extremely low solubility.

**Use of Polymeric, Phosphorus-Containing Ligands in Catalyst Compositions**

A further aspect of the present invention provides a catalyst composition comprising at least one of the polymeric ligand compositions of the present invention combined with a Group VIII transition metal, transition metal compound, transition metal complex, or combinations thereof and, optionally a Lewis Acid. Generally, any Group VIII metal or metal compound can be used to combine with the composition. The term "Group VIII" refers to the ACS version of the Periodic Table of the Elements, "CRC Handbook of Chemistry and Physics", 67$^{th}$ edition, Boca Raton, Florida: CRC Press, 1986-1987.

Generally, a Group VIII metal or compound thereof is combined with at least one polymeric ligand of the present invention to provide the catalyst. Among the Group VIII metal compounds, nickel, cobalt, and palladium compounds are preferred for hydrocyanation catalysts. A nickel compound is more preferred. A zero-valent nickel compound containing a ligand that can be displaced by the polymeric ligand of the present invention is the most preferred source of Group VIII metal or Group VIII metal compound. Zero-valent nickel compounds can be prepared or generated according to methods known in the art, such as those described in U. S. Pat. Nos. 3,496,217; 3,631,191; 3,846,461; 3,847,959 and 3,903,120, incorporated herein by reference. Three preferred zero-valent nickel compounds are $Ni(COD)_2$ (COD is 1,5-cyclooctadiene), $Ni(P(O\text{-}o\text{-}C_6H_4CH_3)_3)_3$ and $Ni\{P(O\text{-}o\text{-}C_6H_4CH_3)_3\}_2(C_2H_4)$, as known in the art.

Alternatively, divalent nickel compounds can be combined with a reducing agent to serve as a source of zero-valent nickel in the reaction. Suitable divalent nickel compounds include compounds of the formula $NiZ^2_2$ where $Z^2$ is halide, carboxylate, or acetylacetonate. Suitable reducing agents include metal borohydrides, metal aluminum hydrides, metal alkyls, Li, Na, K, or $H_2$. Elemental nickel, preferably nickel powder, when combined with a halogenated catalyst, as described in U. S. Pat. No. 3,903,120 (incorporated herein by reference) is also a suitable source of zero-valent nickel.

The chelating arrangement of donor atoms in bidentate ligands results in a strong ligand-metal interaction and thus greatly minimizes the potential for metal leaching. It is possible to alter the spacing between the chelating atoms, the steric environment of these atoms, and the electronic properties of the donor atoms, offering control of ligand coordination properties thereby optimizing catalyst performance.

**Hydrocyanation using Polymeric, Phosphorus-Containing Ligands**

According to a further aspect of the present invention, at least one of the polymeric ligand compositions of the present invention may be used to form a catalyst (with or without a Lewis acid) which may be used for the hydrocyanation of organic compounds. The process comprises contacting, in the presence of the catalyst, an unsaturated organic compound with a hydrogen cyanide-containing fluid under conditions sufficient to produce a nitrile, wherein the catalyst comprises a Group VIII metal, at least one of the polymeric ligands described above, and optionally a Lewis acid. The term "fluid" may be gas, liquid, or both. Any fluid containing about 1 to 100 % HCN can be used. Preferably, the HCN contains less than 10 ppm CO, less than 20 ppm cyanogen, less than 10 ppm epoxide, less than 20 ppm acrylonitrile, less than 20 ppm sulfur dioxide, less than 40 ppm sulfuric acid, and less than 100 ppm peroxides. Pure hydrogen cyanide may be used.

The hydrocyanation process may be carried out, for example, by charging a suitable vessel such as a reactor with an unsaturated compound, catalyst composition, and solvent, if any, to form a reaction mixture. Hydrogen cyanide can be initially combined with other components to form the mixture. However, it is preferred that HCN be added slowly to the mixture after the other components have been combined. Hydrogen cyanide may be delivered as a liquid or as a vapor to the reaction. As an alternative, a cyanohydrin may be used as the source of HCN, as in, for example, U.S. Pat. No. 3,655,723.

Another suitable technique is to charge the vessel with the catalyst and the solvent (if any) to be used, and feed both the unsaturated compound and the HCN slowly to the reaction mixture.

The molar ratio of unsaturated compound to catalyst can be varied from about 10:1 to about 100,000:1. The molar ratio of HCN to catalyst generally is varied from about 10:1 to 100,000:1, preferably 100:1 to 5,000:1, for a batch operation. In a continuous operation, such as when using a fixed bed catalyst type of operation, a higher proportion of catalyst can be used such as 5:1 to 100,000:1, preferably 100:1 to 5,000:1, HCN to catalyst.

Preferably, the reaction mixture is agitated; for example, by stirring or shaking. The reaction may be run either batchwise or continuously. The reaction product may be recovered by conventional techniques such as distillation.

The hydrocyanation may be carried out with or without a solvent. The solvent, if used, may be liquid at the reaction temperature and pressure and inert towards the olefin and the catalyst. Suitable solvents include hydrocarbons such as benzene, xylene, or combinations thereof; ethers, such as tetrahydrofuran (THF); nitriles, such as acetonitrile, benzonitrile, or adiponitrile, or combinations of two or more thereof. The unsaturated compound to be hydrocyanated may itself serve as the solvent. Hydrocyanation may also be carried out in the gas phase.

The exact temperature is dependent to a certain extent on the particular catalyst being used, the particular unsaturated compound being used and the desired reaction rate. Normally, temperatures of from -25°C to 200°C may be used, the range of 0°C to 150°C being preferred.

Atmospheric pressure is satisfactory for carrying out the reaction, and pressures of from about 0.05 to 10 atmospheres (50.6 to 1013 kPa) are preferred. Higher pressures, up to 10,000 kPa or more, may be used, if desired, but any benefit that may be obtained thereby would need to justify the increased cost of such operations.

The time required may be in the range of from a few seconds to many hours (such as 2 seconds to 24 hours), depending on the particular conditions and method of operation.

The unsaturated compound has 2 to about 30 carbon atoms per molecule and can be cyclic or acyclic. It can have the formula of $R^8CH=CH-CH=CR^9$, $CH=CH-(CH_2)_q-R^{10}$, $CH_3-(CH_2)_n-CH=CH-(CH_2)_q-R^{10}$, and combinations of two or more thereof in which $R^8$ and $R^9$ are each independently H, $C_1$ to $C_3$ alkyl, or combinations thereof; $R^{10}$ is H, CN, $CO_2R^{11}$, or perfluoroalkyl having 1 to about 20 carbon atoms; n is an integer of 0 to 12; q is an integer of 0 to 12 when $R^{10}$ is H, $CO_2R^{11}$ or perfluoroalkyl; q is an integer of 1 to 12 wherein $R^{10}$ is CN; and $R^{11}$ is $C_1$ to $C_{12}$ alkyl or cycloalkyl, $C_6$ to $C_{20}$ aryl, or combinations thereof.

The unsaturated compound can be an acyclic, aliphatic, monoethylenically unsaturated compound or cyclic monoethylenically unsaturated compound, or combinations of two or more thereof. Nonlimiting examples of ethylenically unsaturated compounds are shown in Formulas V and VI, and the corresponding terminal nitrile compounds produced are illustrated by Formulas VII and VIII, respectively, wherein numerically designated radicals have the same meaning and where $R^9$ is the same as disclosed above.

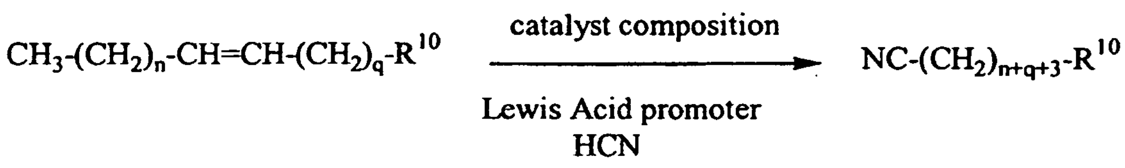


Formula V Formula VII

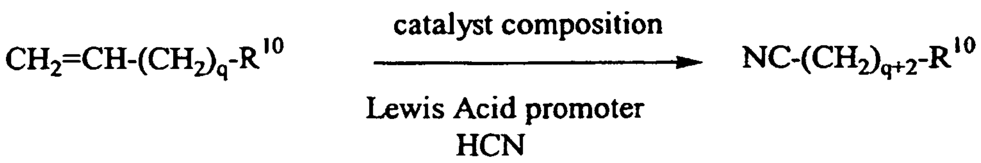


Formula VI Formula VIII

Preferably, the unsaturated organic compounds contain less than 100 ppm peroxides. Examples of suitable ethylenically unsaturated compounds include ethylene, propylene, 1-butene, 2-pentene, 2-hexene, cyclohexene, cyctopentene, allene, 3-pentenenitrile, 4-pentenenitrile, methyl 3-pentenoate, $C_bF_{2b+1}$, where b is an integer of up to 20 and combinations of two or more thereof. The monoethylenically unsaturated compounds can also be conjugated to an ester group such as methyl 2-pentenoate. Preferred olefins are linear alkenes, linear alkenenitriles, linear alkenoates, linear 2-alkenoates, perfluoroalkyl ethylenes, and combinations of two or more thereof. Most preferred substrates include 3- and 4-pentenenitrile, alkyl 2-, 3-, and 4-pentenoates, and $C_bF_{2b+1}CH=CH_2$ (where b is 1 to 12), and combinations of two or more thereof. 3-pentenenitrile and 4-pentenenitrile are especially preferred olefins.

When non-conjugated acyclic aliphatic monoethylenically unsaturated compounds are used, up to about 10% by weight of the monoethylenically unsaturated compound can be present in the form of a conjugated isomer, which itself may undergo hydrocyanation. For example, when 3-pentenenitrile is used, as much as 10% by weight thereof may be 2-pentenenitrile (as used herein, the term "pentenenitrile" is intended to be identical with "cyanobutene"). Suitable unsaturated compounds include unsubstituted hydrocarbons, as well as hydrocarbons substituted with groups that do not attack the catalyst, such as the cyano group.

The process of this invention can be carried out in the presence of one or more Lewis acid promoters that affect both the activity and the selectivity of the catalyst system. The promoter may be an inorganic or organometallic compound in which the cation is selected from scandium, titanium, vanadium, chromium, manganese, iron, cobalt, copper, zinc, boron, aluminum, yttrium, zirconium, niobium, molybdenum, cadmium, rhenium, lanthanum, europium, ytterbium, tantalum, and samarium, and tin. Examples include $ZnBr_2$, $ZnI_2$, $ZnCl_2$, $ZnSO_4$, $CuCl_2$, CuCl, $Cu(O_3SCF_3)_2$, $CoCl_2$, $CoI_2$, $FeI_2$, $FeCl_3$, $FeCl_2$, $FeCl_2(THF)_2$, $TiCl_4(THF)_2$, $TiCl_2$, $ClTi(OiPr)_2$, $MnCl_2$, $ScCl_3$, $AlCl_3$, $(C_8H_{17})AlCl_2$, $(C_8H_{17})_2AlCl$, (iso-$C_4H_9)_2AlCl$, $Ph_2AlCl$, $PhAlCl_2$, $ReCl_5$, $ZrCl_4$, $NbCl_5$, $VCl_3$, $CrCl_2$, $MoCl_5$, $YCl_3$, $CdCl_2$, $LaCl_3$, $Er(O_3SCF_3)_3$, $Yb(O_2CCF_3)_3$, $SmCl_3$, $(C_6H_5)_3SnX$, where $X=CF_3SO_3$, $CH_3C_6H_5SO_3$, or $(C_6H_5)_3BCN$, $B(C_6H_5)_3$, and $TaCl_5$. Suitable promoters are further described in U. S. Pat. Nos. 3,496,217; 3,496,218 and 4,774,353. These include metal salts (such as $ZnCl_2$, $CoI_2$, and $SnCl_2$), and organometallic compounds (such as $R^{12}AlCl_2$, $R^{12}SnO_3SCF_3$, and $R^{12}B$, where $R^{12}$ is an alkyl or aryl group). U.S. Pat. No. 4,874,884 describes how synergistic combinations of promoters can be chosen to increase the catalytic activity of the catalyst system. Preferred promoters include $CdCl_2$, $FeCl_2$, $ZnCl_2$, $B(C_6H_5)_3$, and $(C_6H_5)_3Sn(CF_3SO_3)$, $CH_3C_6H_5SO_3$, or $(C_6H_5)_3BCN$. The mole ratio of promoter to Group VIII transition metal present in the reaction can be within the range of about 1:16 to about 50:1.

Hydrocyanation can also be carried out with a conjugated, unsaturated compound. With a conjugated, unsaturated compound, a Lewis Acid promoter is optional. Examples of conjugated, unsaturated compounds containing from about 4 to about 15, preferably 4 to 10 carbon atoms are 1,3 -butadiene, *cis* and *trans*-2,4-hexadienes, *cis* and *trans*-1,3-pentadienes, 1,3-cyclooctadiene and combinations of two or more thereof. Butadiene is especially preferred by reason of its commercial importance in the production of adiponitrile. Preferably, the butadiene contains less than 5 ppm t-butyl catechol, less than 500 ppm vinylcyclohexene, and less than 100 ppm peroxides.

The following Formulas IX and X illustrate some suitable starting conjugated olefins.

$CH_2=CH-CH=CH_2$ $R^{13}CH=CH-CH=CHR^{14}$

1,3-butadiene

IX -X

wherein each of $R^{13}$ and $R^{14}$, independently, is H or a $C_1$ to $C_3$ alkyl. Formulas XI, XII, and XIII represent the products obtained from 1,3-butadiene and HCN:

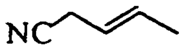
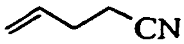
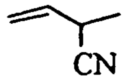

XI XII XIII

3PN 4PN

2M3BN

where 3PN is 3-pentenenitrile, 4PN is 4-pentenenitrile, and 2M3BN is 2-methyl-3-butenenitrile.

The reaction of a conjugated unsaturated compound and a HCN-containing fluid can be carried out in the same manner as that described above in relation to monoethylenically unsaturated compounds.

**Isomerization Using Polymeric, Phosphorus-Containing Ligands**

In a further aspect of the present invention, the polymeric ligand compositions of the present invention may be used to form catalysts, which may be used for the isomerization of branched nitriles to linear nitriles. The isomerization comprises contacting an alkenyl nitrile with a catalyst disclosed above under conditions sufficient to isomerize the alkenyl nitrile. The process can be run with or without a Lewis acid. Examples of suitable alkenyl nitriles include, but are not limited to, 2-alkyl-3-monoalkenenitriles, 3-alkenenitriles, or combinations thereof. The alkenyl nitrile may be produced by a batch or continuous hydrocyanation process. The isomerization can be carried out under substantially similar conditions as described above in relation to hydrocyanation. Preferably, the branched nitriles contain less than 100 ppm peroxides.

A 2-alkyl-3-monoalkenenitrile used as the starting material in the isomerization can be made by the hydrocyanation of a diolefin as described above or can come from any other available sources. The olefinic double bond in the 2-alkyl-3-monoalkenenitriles used as starting materials in the isomerization cannot be conjugated to the triple bond of the cyano group. Suitable starting 2-alkyl-3-monoalkenenitriles can also carry groups that do not attack the catalyst, including, for example, another cyano group. Preferably, the starting 2-alkyl-3-monoalkenenitriles contain from 5 to 8 carbon atoms, excluding any additional substitution. 2-Methyl-3-butenenitrile is an especially important starting material, because it is used to produce adiponitrile. Other representative nitrile starting materials include 2-ethyl-3-butenenitrile and 2-propyl-3-butenenitrile.

When the starting nitrile is 2-methyl-3-butenenitrile (2M3BN, Formula XIII), the isomerization products are those shown in Formulas XI and XII. above.

The isomerization process of this invention can be carried out, for example, at atmospheric pressure and at any temperature in the range of 10 to 200°C, preferably in the range of 60 to 150°C. The pressure is not critical, however, and can be above or below atmospheric pressure, if desired. Any of the conventional batch or continuous flow procedures may be used either in the liquid phase or in the vapor phase (with respect to the relatively volatile 2-methyl-3-butenenitrile reactant and linear pentenenitrile products). The reactor may be of any mechanically and chemically resistant material, and is usually of glass or an inert metal or alloy, such as nickel, copper, silver, gold, platinum, stainless steel, Monel® metal alloy or Hastelloy® metal alloy.

The process can be carried out in the absence or in the presence of a solvent or diluent. Any solvent or diluent that is inert to, or nondestructive of, the catalyst can be used. Suitable solvents include, but are not limited to, aliphatic or aromatic hydrocarbons (hexane, cyclohexane, benzene), ethers (diethyl ether, tetrahydrofuran, dioxane, glycol dimethyl ether, anisole), esters (ethyl acetate, methyl benzoate, nitriles (acetonitrile, benzonitrile), or combinations of two or more thereof.

The catalyst (complex of Group VIII metal, preferably nickel, and polymeric ligand) is essentially nonvolatile, whereas the 2-methyl-3-butenenitrile reactant and the linear pentenenitrile products are relatively volatile. Accordingly, in a continuous flow procedure, the catalyst can be a component of the flowing system in a slurry-liquid-phase operation.

It can also be in a mobile non-flowing liquid state in a semi-vapor phase operation, or it may be in a fixed-bed state in a conventional flowing vapor-phase operation or flowing liquid-phase operation.

The time required for the isomerization process to obtain a practical level of conversion of, for example, 2-alkyl-3-monoalkenenitrile, to linear alkenenitrile is dependent upon the temperature of reaction, i.e., operation at lower temperature generally requires a longer time than operation at a higher temperature. A practical reaction time can be in the range of a few seconds to many hours (2 seconds to about 24 hours), depending on the particular conditions and method of operation.

The molar ratio of 2-alkyl-3-monoalkenenitrile to catalyst is generally greater than 1:1, usually in the range from about 5:1 to 20,000:1, preferably 100:1 to 5,000:1, for a batch or continuous operation.

## Hydroformylation using Polymeric, Phosphorus-Containing Ligands

In a further aspect of the present invention, the polymeric ligands of the present invention may be used to form catalysts which may be used for hydroformylation of monoethylenically unsaturated organic compounds with 2 to 20 carbon atoms to produce corresponding aldehydes. The catalyst comprises a Group VIII metal or Group VIII metal compound combined with at least one polymeric ligand of the present invention. Preferred Group VIII metals for hydroformylation reactions are rhodium, iridium, and platinum, the most preferred being rhodium. The Group VIII metal may be in the form of a compound, such as a hydride, halide, organic acid salt, ketonate, inorganic acid salt, oxide, carbonyl compound, amine compound, or combinations of two or more thereof. Preferred Group VIII metal compounds are $Ir_4(CO)_{12}$, $IrSO_4$, $RhCl_3$, $Rh(NO_3)_3$. $Rh(OAc)_3$, $Rh_2O_3$, $Rh(acac)(CO)_2$, $[Rh(OAc)(COD)]_2$, $Rh_4(CO)_{12}$, $Rh_6(CO)_{16}$, $RhH(CO)(Ph_3P)_3$, $[Rh(OAc)(CO)_2]_2$, $[RhCl(COD)]_2$, and combinations of two or more thereof ("acac" is an acetylacetonate group; "OAc" is an acetyl group; "COD" is 1,5-cyclooctadiene; and "Ph" is a phenyl group). However, it should be noted that the Group VIII metal compounds are not necessarily limited to the above listed compounds. Rhodium compounds suitable for hydroformylation can be prepared or generated according to techniques well known in the art, as described, for example, in PCT Pat. App. WO 9530680, U. S. Pat. No. 3,907,847, and J. Am. Chem. Soc., 1993, 115, 2066. Rhodium compounds that contain ligands that can be displaced by the present polymeric phosphite ligands are a preferred source of rhodium. Examples of such preferred rhodium compounds are $Rh(CO)_2$ (acac), $Rh(CO)_2(C_4H_9COCHCO\text{-}t\text{-}C_4H_9)$, $Rh_2O_3$, $Rh_4(CO)_{12}$, $Rh_6(CO)_{16}$, $Rh(O_2CCH_3)_2$, Rh(2-ethylhexanoate), and combinations of two or more thereof.

The amount of transition metal in the catalyst may be varied and may be determined by balancing catalyst activity and process economy. In general, the molar ratio of polymeric ligand to transition metal generally can be from about 1:1 to about 100:1, preferably from about 2:1 to about 20:1, moles phosphorus per mole metal.

The reactant of the hydroformylation process is an unsaturated organic compound having at least one "C=C" bond in the molecule and preferably 2 to about 20 carbon atoms. Examples of suitable ethylenically unsaturated organic compounds include, but are not limited to, linear terminal olefinic hydrocarbons (i.e., ethylene, propylene, 1-butene, 1-pentene, 1-hexene, 1-octene, 1-nonene, 1-decene, 1-tetradecene, 1-hexadecene, 1-octadecene, 1-eicosene and 1-dodecene); branched terminal olefinic hydrocarbons (i.e., isobutene and 2-methyl-1-butene); linear internal olefinic hydrocarbons (i.e., *cis*- and *trans*-2-butene, *cis*- and *trans* -2-hexene, *cis*- and *trans* -2-octene, and *cis*- and *trans* -3-octene); branched internal olefinic hydrocarbons (2,3-dimethyl-2-butene, 2-methyl-2-butene and 2-methyl-2-pentene); terminal olefinic hydrocarbons; internal olefinic hydrocarbon mixtures (i.e., octenes, prepared by dimerization of butenes); cyclic olefins (i.e., cyclohexene, and cyclooctene); and combinations of two or more thereof. Preferably, the unsaturated organic compound contains less than 100 ppm peroxides.

Examples of suitable olefinic compounds also include those substituted with an unsaturated hydrocarbon group, including olefinic compounds containing an aromatic substituent such as styrene, alphamethylstyrene and allylbenzene.

The unsaturated organic compound may also be substituted with one or more functional groups containing a heteroatom, such as oxygen, sulfur, nitrogen or phosphorus. Examples of these heteroatom-substituted, ethylenically unsaturated organic compounds include vinyl methyl ether, methyl oleate, oleyl alcohol, 3-pentenenitrile, 4-pentenenitrile, 3-pentenoic acid, 4-pentenoic acid, methyl 3-pentenoate, 7-octen-1-al, acrylonitrile, acrylic acid esters, methyl acrylate, methacrylic acid esters, methyl methacrylate, acrolein, allyl alcohol, 3-pentenal, 4-pentenal, and combinations of two or more thereof.

The hydroformylation process of the invention can be illustrated as follows:

$$CH_3-(CH_2)_p-CH=CH-(CH_2)_r-R^{15} \xrightarrow[H_2 / CO]{\text{catalyst composition}} OHC-(CH_2)_{p+r+3}-R^{15}$$

Formula XIV                Formula XV

$$CH_2=CH-(CH_2)_r-R^{15} \xrightarrow[H_2 / CO]{\text{catalyst composition}} OHC-(CH_2)_{r+2}-R^{15}$$

Formula XVI                Formula XVII

In the above equations, $R^{15}$ is H, -CN, $-CO_2R^{16}$, $-C(O)N(R^{16})_2$, - CHO, $-OR^{16}$, OH, or combinations of two or more thereof; p is an integer from 0 to 12; and r is an integer from 0 to 12. Each $R^{16}$ is independently selected from the group consisting of H, $C_1$ to $C_{20}$ branched or straight chain alkyl, $C_1$ to $C_{20}$ cycloalkyl, and $C_6$ to $C_{20}$ aryl.

Particularly preferred unsaturated organic compounds are 3-pentenenitrile, 3-pentenoic acid, 3-pentenal, allyl alcohol, and alkyl 3-pentenoate, such as methyl 3-pentenoate, and combinations of two or more thereof. Preferably, the 3-pentenenitrile, 3-pentenoic acid, 3-pentenal, allyl alcohol, and alkyl 3-pentenoate contain less than 100 ppm peroxides. The linear aldehyde compound prepared by the present process starting with one of these compounds can be used advantageously in the preparation of ε-caprolactam, hexamethylenediamine, 6-aminocaproic acid, 6-aminocapronitrile or adipic acid, which are precursors for nylon-6 and/or nylon-6,6.

The hydroformylation process of the invention also can be carried out with a mixture that comprises two or more unsaturated organic compounds. For example, 3-pentenenitrile can be present in a mixture containing 4-pentenenitrile. Because the 4- isomer reacts in a similar fashion as the corresponding 3-isomer to the desired linear aldehyde, a mixture of isomers can be used directly in the present process.

The 3-pentenenitrile may be present in mixtures containing impurities that do not interfere with the hydroformylation reaction. An example of such an impurity is 2-pentenenitrile.

The hydroformylation process of the invention can be carried out by any means known to one skilled in the art, such as, for example, the one disclosed in U. S. Pat. No. 4,769,498. Generally, the process can be carried out under any condition sufficient to effect the production of a desired aldehyde. For example, the temperature can be from about 0°C to 200°C, preferably from about 50 to 150°C, and more preferably from 85° to 110°C. The pressure may vary from atmospheric pressure to 5 MPa, preferably from 0.1 to 2 MPa. The pressure is, as a rule, equal to the combined hydrogen and carbon monoxide partial pressures. Inert gases also may be present; the pressure may vary from atmospheric pressure to 15 MPa when inert gases are present. The molar ratio of hydrogen to carbon monoxide is generally between 10:1 and 1 :10, and preferably between 6:1 and 1:2 moles hydrogen/mole carbon monoxide. It is most preferred that a 1:1 ratio of carbon monoxide to hydrogen is used.

The amount of catalyst is selected so that favorable results can be obtained with respect to catalyst activity and process economy. In general, the amount of transition metal in the reaction, which comprises an unsaturated organic compound, a catalyst composition, and solvent (if present), can be between 10 and 10,000 ppm and more preferably between 50 and 1,000 ppm, calculated as free metal.

The solvent may be the mixture of reactants of the hydroformylation reaction itself, such as the starting unsaturated compound, the aldehyde product and/or by-products. Other suitable solvents include saturated hydrocarbons (for example, kerosene, mineral oil, or cyclohexane), ethers (for example, diphenyl ether or tetrahydrofuran), ketones (for example, acetone, cyclohexanone), nitriles (for example, acetonitrile, adiponitrile or benzonitrile), aromatics (for example, toluene, benzene, or xylene), esters (for example, methyl valerate, caprolactone), dimethylformamide, or combinations of two or more thereof.

The hydroformylation process can be run in solution or in the gas phase. When the hydroformylation is carried out in the vapor phase, the preferred temperature range is from about 50°C to about 180°C, most preferably from about 90°C to 110°C. The temperature must be chosen high enough to maintain all of the reactants and products in the vapor

phase, but low enough to prevent deterioration of the catalyst. The particular preferred temperature depends to some extent on the catalyst being used, the olefinic compound being used, and the desired reaction rate. The operating pressure is not particularly critical and can be from about 0.1 to 1.0 MPa. The pressure and temperature combination must be chosen so as to maintain reactants and products in the vapor phase. A given catalyst is loaded into a reactor, such as a tubular reactor, taking care to avoid exposure of oxygen-sensitive catalysts to oxygen from the air. A gaseous mixture of the desired olefinic compound, carbon monoxide and hydrogen, along with any desired diluent, such as nitrogen, helium or argon, is then passed through the reactor while contacting the catalyst. The reaction products are generally liquid at room temperature and are conveniently recovered by cooling. The reactor effluent can be directly connected to a sampling valve and can be analyzed by gas chromatography. Aldehydic products, such as linear and branched butyraldehydes obtained from hydroformylation of propylene, can be quantitatively separated and analyzed using a 30M DB-Wax® capillary GC column.

For the hydrocyanation, isomerization, and hydroformylation processes described herein, a non-oxidizing environment is desirable in order to retard oxidative deactivation of the catalyst. Accordingly, an inert atmosphere, e.g., nitrogen, is preferably used, although air can be used, if desired, at the expense of loss of a proportion of the catalyst activity through oxidation. Impurities that are detrimental to the catalyst should be kept to a minimum.

The following examples are presented to further illustrate specific features and embodiments of the present invention including various methods of preparing the polymeric substrate on which the phosphorus-containing ligand is to be formed and methods of forming the phosphorus-containing ligand compositions. Similarly the specific reactions and compounds when identified structurally by chemical formula are intended to be illustrative of the reaction pathway for the formation of phosphorus-containing bidentate ligand compositions of interest. As such, it should be appreciated that other species and distribution of products will be present as generally known in the art and that any performance data associated with such compositions was derived using the mixture as produced without isolation or separation of the specific compound. All parts, proportions, and percentages are by weight, unless otherwise indicated.

In evaluating the performance of the respective phosphorus-containing ligand compositions, the following general procedures were employed unless otherwise noted:

*Preparation of catalyst:* A catalyst solution is prepared by adding 0.0039 grams of $Ni(COD)_2$ (0.014 mmol) dissolved in 0.320 mL toluene to a specified quantity of the respective phosphorus-containing polymeric ligand composition being evaluated dissolved in 0.200 mL THF.

*Hydrocyanation of butadiene:* A specified volume of the above catalyst solution nominally containing about 0.0020 mmol Ni is added to each of 2 reaction vials fitted with septum caps. The vials are cooled to - 20°C and 120 μL of a solution of HCN in valeronitrile (0.830 mmol HCN) and 280 μL of a solution of butadiene in toluene (0.925 mmol BD) are added to each vial. The vials are sealed and placed in a hot block reactor set at 80°C. Samples are removed after 1.5 and 3 hours and quenched by cooling to -20°C. The reaction mixtures are then diluted in ethyl ether and the product distribution analyzed by GC against valeronitrile as an internal standard. The results are presented as relative percent of the starting HCN that had been converted to useful nitriles (3-pentenenitrile (3PN), and 2-methyl-3-butenenitrile (2M3BN)).

*Isomerization of 2-methyl-3-butene nitrile (2M3BN):* A specified volume of the above catalyst solution containing nominally about 0.002 mmol Ni is added to 2 reaction vials fitted with septum caps. 130 μL of a cold solution containing 2M3BN and valeronitrile (0.930 mmol 2M3BN) are added to each vial. The vials are sealed and placed in a hot block reactor set at 125°C. Samples are removed after 1.5 and 3 hrs, cooled and diluted in ethyl ether. GC using valeronitrile as an internal standard is employed to analyze the product distribution. The results are presented as the 3PN/2M3BN ratio.

*Hydrocyanation of 3-pentenenitrile (3PN):* A specified volume of the above catalyst solution nominally containing about 0.003 mmol Ni and 13 μL of a solution of $ZnCl_2$ in 3PN (0.0067 mmol $ZnCl_2$) are added to a vial fitted with a septum cap. The vial is cooled to -20°C and 125 μL of a solution of HCN, 3PN, and 2-ethoxyethyl ether (0.396 mmol HCN, 0.99 mmol 3PN) are added. The 3PN used for hydrocyanation and hydroformylation container approximately 97% *t*-3-pentenenitrile (GC). The vial is sealed and set aside for 24 hours at room temperature. The reaction mixture is diluted with ethyl ether and the product distribution is analyzed by GC using 2-ethoxyethyl ether as an internal standard. The results are presented as the relative percent of the starting pentenenitriles that have been converted to dinitrile product and the percent yield based on HCN. The selectivity to the linear adiponitrile (ADN) isomer is reported as percent ADN in reaction product mixture.

In some examples of hydrocyanation of 3-pentenenitrile, the HCN was added batchwise at the beginning of the experiment and the mixture was heated in a hot block reactor. In some examples, the HCN was added slowly over the course of the experiment and the mixtures were heated in a thermostatically controlled oil bath. HCN was delivered to the flask as an $HCN/N_2$ gas mixture by bubbling dry nitrogen gas through liquid HCN at 0°C (maintained in a 0°C circulating bath), providing a vapor stream which is about 35% HCN (vol/vol). The rate of nitrogen gas flow determines

the rate of HCN delivery. Samples were periodically analyzed by gas chromatography (GC).

## EXAMPLES

### Example 1

Polymer derived from the oxidative coupling of 4,4'-ethylidenebis(2-isopropyl-5-methyl)phenol:

To a 25 mL vial with a magnetic stirrer was added 1.0 g of 4,4'-ethylidenebis(2-isopropyl-5-methyl)phenol (prepared as described in Bull. Chem. Soc. Jpn., 1989, 62, 3603) and 50 mg of Cu(OH)Cl(TMEDA), where TMEDA is tetramethytethylenediamine, prepared according to Tetrahedron Lett., 1994, 35, 7983, and 3 mL of methylene chloride. The mixture was stirred overnight, an additional 6 mL of methylene chloride was added, and the mixture was stirred for two more days. The mixture was treated with an aqueous solution of sodium EDTA (ethylenediaminetetraacetic acid). The aqueous layer was removed and the solid filtered and washed with acetone. After drying under vacuum, 0.833 g of a brown solid was obtained.

### Example 1A

Preparation of polymeric phosphite ligand from the reaction of polymer with the phosphorochloridite of o-cresol:

A 100 mL round bottom flask with a magnetic stir bar was charged with 490 mg of the brown solid polymer described in Example 1 and 841 mg of the phosphorochlorodite of o-cresol in about 30 mL of tetrahydrofuran (THF). About 1 g of tri-n-butylamine was added. The mixture was stirred overnight and the solvent removed under vacuum. Acetonitrile was added and the solid was filtered, washed with acetonitrile and vacuum dried to give 713 mg of a yellow solid.

### Example 1B

Preparation and use of catalyst with polymer from Example 1 A:

*Preparation of catalyst:* 0.034 g of the polymeric phosphite ligand described in Example 1A was weighed into a reaction vial equipped with a septum cap. 200 μL of THF were added to the vial and the sample was shaken. A catalyst solution was prepared by adding 0.0039 g of $Ni(COD)_2$ (0.014 mmol) in 0.320 mL toluene to the vial.

*Hydrocyanation of butadiene:* A catalyst sample prepared as above was cooled to -20°C and 280 L of a solution of butadiene in toluene (0.925 mmol butadiene) and 120 L of a solution of HCN in valeronitrile (0.830 mmol HCN) were added. The mixture was heated at 80°C. After 3 hours, a sample was removed and quenched by cooling to -20°C. The mixture was then diluted in ethyl ether and the product distribution analyzed by GC against valeronitrile as internal standard. Analysis showed that 64% of the starting HCN had been converted to useful nitriles (the 3-pentenenitrile/2-methyl-3-butenenitrile, 3PN/2M3BN, ratio was 16.3).

*Hydrocyanation of 3-pentenenitrile (3PN):* Another catalyst sample prepared as above was cooled to -20°C and 125 μL of a solution of HCN, 3PN, and 2-ethoxyethyl ether (0.396 mmol HCN, 0.99 mmol 3PN) were added. 13 μL of a solution of $ZnCl_2$ in 3PN (0.0067 mmol $ZnCl_2$) were added to the vial. The vial was sealed and set aside for 24 hours at room temperature. The reaction mixture was diluted with ethylether and the product distribution analyzed by GC using 2-ethoxyethyl ether as an internal standard. Analysis showed that 66% of the starting HCN had been converted to dinitrile product. The selectivity to the linear ADN isomer was 97.6%.

*Aspect 9: Isomerization of 2-methyl-3-butenenitrile (2M3BN):* To another catalyst sample prepared as above was added 130 L of a cold solution containing 2M3BN (0.930 mmol) and valeronitrile. The mixture was heated to 120°C for 3 hours. GC analysis with valeronitrile as an internal standard indicated that a 3PN/2M3BN ratio of 19.2 had been reached.

Having thus described and exemplified the invention with a certain degree of particularity, it should be appreciated that the following claims are not to be so limited but are to be afforded a scope commensurate with the wording of each element of the claim and equivalents thereof.

## Claims

**1.** A process for preparing a polymeric, phosphorus-containing composition by:

(1) aryl to aryl oxidative coupling of monomer having the structure:

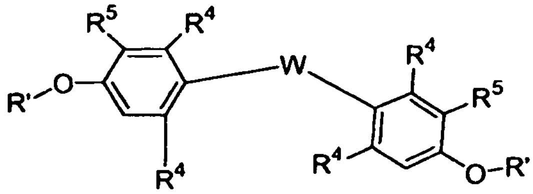

wherein:

W is selected from $C_6$-$C_{20}$ arylene, a $C_1$-$C_{20}$ alkylene and cycloalkylene;
each R' is individually hydrogen or a hydroxyl protective group selected from, but not limited to, alkyl, alkoxyalkyl, carbonylalkyl, or a crown ether formed by taking both R' groups together;
each $R^4$ is independently H, $C_1$ to $C_{20}$ branched or straight chain alkyl, $C_1$ to $C_{20}$ cycloalkyl, or $C_6$ to $C_{20}$ aryl;
each $R^5$ is independently $C_1$ to $C_{20}$ branched or straight chain alkyl, $C_1$ to $C_{20}$ cycloalkyl, or $C_6$ to $C_{20}$ aryl; and

(2) converting R' with a substituent selected from -P(-O-Ar)$_2$, -P(Ar)$_2$, and - P(Ar)(-O-Ar), where each Ar is individually phenyl, substituted phenyl, naphthyl, or substituted naphthyl, provided that the two Ar groups that are directly or indirectly bonded to the same phosphorus atom may be linked to each other by a linking unit selected from direct bond, alkylidene, secondary or tertiary amine, oxygen, sulfide, sulfone, and sulfoxide; and each Ar can be further substituted with $C_1$ to $C_{20}$ branched or straight chain alkyl, $C_1$ to $C_{20}$ cycloalkyl, $C_6$ to $C_{20}$ aryl, acetal, ketal, cycloalkoxy, aryloxy, perhaloalkyl, fluorine, chlorine, bromine, formyl, ester, hydrocarbylsulfinyl, hydrocarbylsulfonyl, hydrocarbylcarbonyl, cyclic ether, $-OR^3$, $-CO_2R^3$, $-SO_3R^3$, $-S(O)R^3$, $-SO_2R^3$, -CHO, - $C(O)R^3$, and CN; where each $R^3$ is independently $C_1$ to $C_{20}$ branched or straight chain alkyl, and $C_1$ to $C_{20}$ cycloalkyl, or $C_6$ to $C_{20}$ aryl.

**2.** A polymeric, phosphorus-containing composition obtainable by (1) aryl to aryl oxidative coupling of monomer, said monomer having the structure:

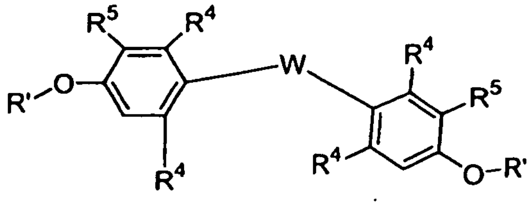

wherein:

W is selected from $C_6$-$C_{20}$ arylene, a $C_1$-$C_{20}$ alkylene and cycloalkylene;
each R' is individually hydrogen or a hydroxyl protective group selected from, but not limited to, alkyl, alkoxyalkyl, carbonylalkyl, or a crown ether formed by taking both R' groups together;
each $R^4$ is independently H, $C_1$ to $C_{20}$ branched or straight chain alkyl, $C_1$ to $C_{20}$ cycloalkyl, or $C_6$ to $C_{20}$ aryl;
each $R^5$ is independently $C_1$ to $C_{20}$ branched or straight chain alkyl, $C_1$ to $C_{20}$ cycloalkyl, or $C_6$ to $C_{20}$ aryl; and

(2) converting R' with a substituent selected from the group consisting of -P(-OAr)$_2$, -P(Ar)$_2$, and -P(Ar)(-O-Ar), wherein each Ar is individually phenyl, substituted phenyl, naphthyl, or substituted naphthyl, provided that the two Ar groups that are directly or indirectly bonded to the same phosphorus atom may be linked to each other by a linking unit selected from direct bond, alkylidene, secondary or tertiary amine, oxygen, sulfide, sulfone, and sulfoxide; and each Ar can be further substituted with $C_1$ to $C_{20}$ branched or straight chain alkyl, $C_1$ to $C_{20}$ cycloalkyl, $C_6$ to $C_{20}$ aryl, acetal, ketal, cycloalkoxy, aryloxy, perhaloalkyl, fluorine, chlorine, bromine, formyl, ester, hydrocarbylsulfinyl, hydrocarbylsulfonyl, hydrocarbylcarbonyl, cyclic ether, $-OR^3$, $-CO_2R^3$, $-SO_3R^3$, $-S(O)R^3$, - $SO_2R^3$, -CHO, $-C(O)R^3$, and CN; where each $R^3$ is independently $C_1$ to $C_{20}$ branched or straight chain alkyl, and $C_1$ to $C_{20}$

cycloalkyl, or $C_6$ to $C_{20}$ aryl.

3. A catalyst composition comprising at least one polymeric, phosphorus-containing composition of Claim 2 and at least one Group VIII metal.

4. The catalyst composition of Claim 3 wherein the Group VIII metal is nickel, palladium, cobalt.

5. The catalyst composition of Claim 3 wherein the Group VIII metal is rhodium, iridium and platinum.

6. The catalyst composition of Claim 4 wherein the Group VIII metal is nickel.

7. The catalyst composition of Claim 4 further comprising a Lewis acid.

8. The catalyst composition of Claim 7 wherein the Lewis acid is selected from the group consisting of $ZnBr_2$, $ZnI_2$, $ZnCl_2$, $ZnSO_4$, $CuCl_2$, CuCl, $Cu(O_3SCF_3)_2$, $CoCl_2$, $CoI_2$, $FeI_2$, $FeCl_3$, $FeCl_2$, $FeCl_2(THF)_2$, $TiCl_4(THF)_2$, $TiCl_2$, ClTi(OiPr)$_2$, $MnCl_2$, $ScCl_3$, $AlCl_3$, $(C_8H_{17})AlCl_2$, $(C_8H_{17})_2AlCl$, (iso-$C_4H_9$)$_2$AlCl, $Ph_2AlCl$, $PhAlCl_2$, $ReCl_5$, $ZrCl_4$, $NbCl_5$, $VCl_3$, $CrCl_2$, $MoCl_5$, $YCl_3$, $CdCl_2$, $LaCl_3$, $Er(O_3SCF_3)_3$, $Yb(O_2CCF_3)_3$, $SmCl_3$, $B(C_6H_5)_3$, $TaCl_5$, and $(C_6H_5)_3SnX$, where X can be $CF_3SO_3$, $CH_3C_6H_5SO_3$, or $(C_6H_5)_3BCN$.

9. The catalyst composition of Claim 8 wherein the Lewis acid is zinc chloride or iron chloride.

10. A hydrocyanation process comprising contacting an unsaturated organic compound with HCN in the presence of a catalyst composition comprising at least one composition of Claim 2 and at least one Group VIII metal, and optionally a Lewis acid.

11. The hydrocyanation process of Claim 10 wherein the Lewis acid is selected from the group consisting of $ZnBr_2$, $ZnI_2$, $ZnCl_2$, $ZnSO_4$, $CuCl_2$, CuCl, $Cu(O_3SCF_3)_2$, $CoCl_2$, $CoI_2$, $FeI_2$, $FeCl_3$, $FeCl_2$, $FeCl_2(THF)_2$, $TiCl_4(THF)_2$, $TiCl_2$, ClTi(OiPr)$_2$, $MnCl_2$, $ScCl_3$, $AlCl_3$, $(C_8H_{17})AlCl_2$, $(C_8H_{17})_2AlCl$, (iso-$C_4H_9$)$_2$AlCl, $Ph_2AlCl$, $PhAlCl_2$, $ReCl_5$, $ZrCl_4$, $NbCl_5$, $VCl_3$, $CrCl_2$, $MoCl_5$, $YCl_3$, $CdCl_2$, $LaCl_3$, $Er(O_3SCF_3)_3$, $Yb(O_2CCF_3)_3$, $SmCl_3$, $B(C_6H_5)_3$, $TaCl_5$, and $(C_6H_5)_3SnX$, where X can be $CF_3SO_3$, $CH_3C_6H_5SO_3$, or $(C_6H_5)_3BCN$.

12. The hydrocyanation process of Claim 11 wherein the Lewis acid is zinc chloride or iron chloride.

13. The hydrocyanation process of Claim 12 wherein the Group VIII metal is nickel, palladium or cobalt.

14. The hydrocyanation process of Claim 13 wherein the unsaturated organic compound is 3-pentenenitrile or 4-pentenenitrile and the Group VIII metal is nickel.

15. The hydrocyanation process of Claim 14 wherein the HCN contains less than 20 ppm sulfur dioxide, less than 40 ppm sulfuric acid, less than 20 ppm cyanogen, less than 10 ppm epoxide, less than 20 ppm acrylonitrile, and less than 100 ppm peroxides, and the pentenenitriles contain less than 100 ppm peroxides.

16. The hydrocyanation process of Claim 15 wherein the Group VIII metal is nickel and the unsaturated organic compound is 1,3-butadiene.

17. The hydrocyanation process of Claim 16 wherein the HCN contains less than 20 ppm sulfur dioxide, less than 40 ppm sulfuric acid, less than 20 ppm cyanogen, less than 10 ppm epoxide, less than 20 ppm acrylonitrile, and less than 100 ppm peroxides, and the 1,3-butadiene contains less than 5 ppm *t*-butyl catechol, less than 500 ppm vinylcyclohexene, and less than 100 ppm peroxides.

18. A hydroformylation process comprising contacting an unsaturated organic compound with CO and $H_2$ in the presence of a catalyst composition comprising at least one composition of Claim 2 and at least one Group VIII metal.

19. The hydroformylation process of Claim 18 wherein the Group VIII metal is rhodium, iridium or platinum.

20. The hydroformylation process of Claim 19 wherein the unsaturated organic compound is selected from the group consisting of 3-pentenenitrile, 3-pentenoic acid, 3-pentenal, allyl alcohol, and alkyl 3-pentenoate or mixture thereof.

**21.** The hydroformylation process of Claim 20 wherein the unsaturated organic compound contain less than 100 ppm peroxides and the Group VIII metal is rhodium.

**22.** An isomerization process comprising reacting an unsaturated organic nitrile compound in the presence of a catalyst composition comprising at least one composition of Claim 2 and at least one Group VIII metal.

**23.** The isomerization process of Claim 22 wherein the Group VIII metal is nickel, palladium or cobalt.

**24.** The isomerization process of Claim 23 wherein the unsaturated organic nitrile compound is 2-methyl-3-butenenitrile and the Group VIII metal is nickel.

**25.** The isomerization process of Claim 24 wherein the 2-methyl-3-butenenitrile contains less than 100 ppm peroxides.

**Patentansprüche**

**1.** Verfahren zum Herstellen einer polymeren, phosphorhaltigen Zusammensetzung durch:

(1) oxidative Aryl-zu-Aryl-Kupplung von Monomer mit der Struktur:

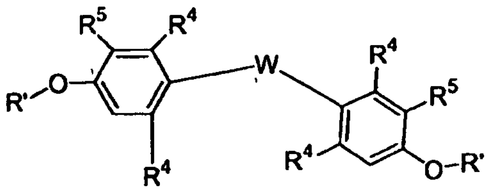

wobei:

W ausgewählt ist aus $C_6$-$C_{20}$-Arylen, einem $C_1$-$C_{20}$-Alkylen und Cycloalkylen;
jedes R' individuell Wasserstoff oder eine Hydroxylschutzgruppe ist, ausgewählt aus, ohne aber darauf beschränkt zu sein, Alkyl, Alkoxyalkyl, Carbonylalkyl oder einem Kronenether, erzeugt durch Zusammennehmen beider R'-Gruppen;
jedes $R^4$ ist unabhängig H, verzweigtes oder geradkettiges $C_1$- bis $C_{20}$-Alkyl, $C_1$- bis $C_{20}$-Cycloalkyl oder $C_6$- bis $C_{20}$-Aryl;
jedes $R^5$ ist unabhängig verzweigtes oder geradkettiges $C_1$- bis $C_{20}$-Alkyl, $C_1$- bis $C_{20}$-Cycloalkyl oder $C_6$- bis $C_{20}$-Aryl; und

(2) Umwandlung von R' mit einem Substituenten, ausgewählt aus $-P(-O-Ar)_2$, $-P(Ar)_2$ und -P(Ar)(-O-Ar), wo jedes Ar individuell Phenyl, substituiertes Phenyl, Naphthyl oder substituiertes Naphthyl ist, mit der Maßgabe, daß die zwei Ar-Gruppen, die direkt oder indirekt an das gleiche Phosphoratom gebunden sind, miteinander durch eine Verknüpfungseinheit, ausgewählt aus direkter Bindung, Alkyliden, sekundärem oder tertiärem Amin, Sauerstoff, Sulfid, Sulfon und Sulfoxid, verknüpft sein können; und jedes Ar weiter mit verzweigtem oder geradkettigem $C_1$- bis $C_{20}$-Alkyl, $C_1$- bis $C_{20}$-Cycloalkyl, $C_6$- bis $C_{20}$-Aryl, Acetal, Ketal, Cycloalkoxy, Aryloxy, Perlialogenalkyl, Fluor, Chlor, Brom, Formyl, Ester, Hydrocarbylsulfinyl, Hydrocarbylsulfonyl, Hydrocarbylcarbonyl, cyclischem Ether, $-OR^3$, $-CO_2R^3$, $-SO_3R^3$, $-S(O)R^3$, $-SO_2R^3$, -CHO, $-C(O)R^3$ und CN substituiert sein kann; wo jedes $R^3$ unabhängig verzweigtes oder geradkettiges $C_1$- bis $C_{20}$-Alkyl und $C_1$- bis $C_{20}$-Cycloalkyl oder $C_6$- bis $C_{20}$-Aryl ist.

**2.** Polymere, phosphorhaltige Zusammensetzung, erhältlich durch (1) oxidative Aryl-zu-Aryl-Kupplung von Monomer, wobei das Monomer folgende Struktur hat:

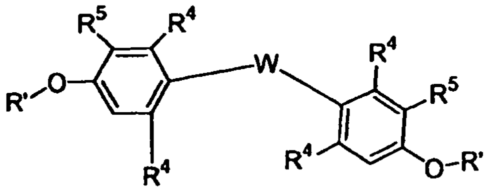

wobei:

W ausgewählt ist aus $C_6$-$C_{20}$-Arylen, einem $C_1$-$C_{20}$-Alkylen und Cycloalkylen;
jedes R' individuell Wasserstoff oder eine Hydroxylschutzgruppe ist, ausgewählt aus, ohne aber darauf beschränkt zu sein, Alkyl, Alkoxyalkyl, Carbonylalkyl oder einem Kronenether, erzeugt durch Zusammennehmen beider R'-Gruppen;
jedes $R^4$ ist unabhängig H, verzweigtes oder geradkettiges $C_1$- bis $C_{20}$-Alkyl, $C_1$- bis $C_{20}$-Cycloalkyl oder $C_6$- bis $C_{20}$-Aryl;
jedes $R^5$ ist unabhängig verzweigtes oder geradkettiges $C_1$- bis $C_{20}$-Alkyl, $C_1$- bis $C_{20}$-Cycloalkyl oder $C_6$- bis $C_{20}$-Aryl; und

(2) Umwandlung von R' mit einem Substituenten, ausgewählt aus der Gruppe, bestehend aus $-P(-O-Ar)_2$, $-P(Ar)_2$ und -P(Ar)(-O-Ar), wobei jedes Ar individuell Phenyl, substituiertes Phenyl, Naphthyl oder substituiertes Naphthyl ist, mit der Maßgabe, daß die zwei Ar-Gruppen, die direkt oder indirekt an das gleiche Phosphoratom gebunden sind, miteinander durch eine Verknüpfungseinheit, ausgewählt aus direkter Bindung, Alkyliden, sekundärem oder tertiärem Amin, Sauerstoff, Sulfid, Sulfon und Sulfoxid, verknüpft sein können; und jedes Ar weiter mit verzweigtem oder geradkettigem $C_1$- bis $C_{20}$-Alkyl, $C_1$- bis $C_{20}$-Cycloalkyl, $C_6$- bis $C_{20}$-Aryl, Acetal, Ketal, Cycloalkoxy, Aryloxy, Perhalogenalkyl, Fluor, Chlor, Brom, Formyl, Ester, Hydrocarbylsulfinyl, Hydrocarbylsulfonyl, Hydrocarbylcarbonyl, cyclischem Ether, $-OR^3$, $-CO_2R^3$, $-SO_3R^3$, - $S(O)R^3$, $-SO_2R^3$, -CHO, $-C(O)R^3$ und CN substituiert sein kann; wo jedes $R^3$ unabhängig verzweigtes oder geradkettiges $C_1$- bis $C_{20}$-Alkyl und $C_1$- bis $C_{20}$-Cycloalkyl oder $C_6$- bis $C_{20}$-Aryl ist.

3. Katalysatorzusammensetzung, umfassend mindestens eine polymere, phosphorhaltige Zusammensetzung nach Anspruch 2 und mindestens ein Metall der Gruppe VIII.

4. Katalysatorzusammensetzung nach Anspruch 3, wobei das Metall der Gruppe VIII Nickel, Palladium, Cobalt ist.

5. Katalysatorzusammensetzung nach Anspruch 3, wobei das Metall der Gruppe VIII Rhodium, Iridium und Platin ist.

6. Katalysatorzusammensetzung nach Anspruch 4, wobei das Metall der Gruppe VIII Nickel ist.

7. Katalysatorzusammensetzung nach Anspruch 4, weiterhin umfassend eine Lewis-Säure.

8. Katalysatorzusammensetzung nach Anspruch 7, wobei die Lewis-Säure aus der Gruppe ausgewählt ist, bestehend aus $ZnBr_2$, $ZnI_2$, $ZnCl_2$, $ZnSO_4$, $CuCl_2$, CuCl, $Cu(O_3SCF_3)_2$, $CoCl_2$, $CoI_2$, $FeI_2$, $FeCl_3$, $FeCl_2$, $FeCl_2(THF)_2$, $TiCl_4(THF)_2$, $TiCl_2$, $ClTi(OiPr)_2$, $MnCl_2$, $ScCl_3$, $AlCl_3$, $(C_8H_{17})AlCl_2$, $(C_8H_{17})_2AlCl$, $(iso\text{-}C_4H_9)_2AlCl$, $Ph_2AlCl$, $PhAlCl_2$, $ReCl_5$, $ZrCl_4$, $NbCl_5$, $VCl_3$, $CrCl_2$, $MoCl_5$, $YCl_3$, $CdCl_2$, $LaCl_3$, $Er(O_3SCF_3)_3$, $Yb(O_2CCF_3)_3$, $SmCl_3$, $B(C_6H_5)_3$, $TaCl_5$ und $(C_6H_5)_3SnX$, wo X $CF_3SO_3$, $CH_3C_6H_5SO_3$ oder $(C_6H_5)_3BCN$ sein kann.

9. Katalysatorzusammensetzung nach Anspruch 8, wobei die Lewis-Säure Zinkchlorid oder Eisenchlorid ist.

10. Hydrocyanierungsverfahren, umfassend Inkontaktbringen einer ungesättigten organischen Verbindung mit HCN in Anwesenheit einer Katalysatorzusammensetzung, umfassend mindestens eine Zusammensetzung nach Anspruch 2 und mindestens ein Metall der Gruppe VIII und gegebenenfalls eine Lewis-Säure.

11. Hydrocyanierungsverfahren nach Anspruch 10, wobei die Lewis-Säure aus der Gruppe ausgewählt ist, bestehend aus $ZnBr_2$, $ZnI_2$, $ZnCl_2$, $ZnSO_4$, $CuCl_2$, CuCl, $Cu(O_3SCF_3)_2$, $CoCl_2$, $CoI_2$, $FeI_2$, $FeCl_3$, $FeCl_2$, $FeCl_2(THF)_2$, $TiCl_4(THF)_2$, $TiCl_2$, $ClTi(OiPr)_2$, $MnCl_2$, $ScCl_3$, $AlCl_3$, $(C_8H_{17})AlCl_2$, $(C_8H_{17})_2AlCl$, $(iso\text{-}C_4H_9)_2AlCl$, $Ph_2AlCl$, $PhAlCl_2$,

$ReCl_5$, $ZrCl_4$, $NbCl_5$, $VCl_3$, $CrCl_2$, $MoCl_5$, $YCl_3$, $CdCl_2$, $LaCl_3$, $Er(O_3SCF_3)_3$, $Yb(O_2CCF_3)_3$, $SmCl_3$, $B(C_6H_5)_3$, $TaCl_5$ und $(C_6H_5)_3SnX$, wo X $CF_3SO_3$, $CH_3C_6H_5SO_3$ oder $(C_6H_5)_3BCN$ sein kann.

**12.** Hydrocyanierungsverfahren nach Anspruch 11, wobei die Lewis-Säure Zinkchlorid oder Eisenchlorid ist.

**13.** Hydrocyanierungsverfahren nach Anspruch 12, wobei das Metall der Gruppe VIII Nickel, Palladium oder Cobalt ist.

**14.** Hydrocyanierungsverfahren nach Anspruch 13, wobei die ungesättigte organische Verbindung 3-Pentennitril oder 4-Pentennitril ist und das Metall der Gruppe VIII Nickel ist.

**15.** Hydrocyanierungsverfahren nach Anspruch 14, wobei das HCN weniger als 20 ppm Schwefeldioxid, weniger als 40 ppm Schwefelsäure, weniger als 20 ppm Cyanogen, weniger als 10 ppm Epoxid, weniger als 20 ppm Acrylnitril und weniger als 100 ppm Peroxide enthält und die Pentennitrile weniger als 100 ppm Peroxide enthalten.

**16.** Hydrocyanierungsverfahren nach Anspruch 15, wobei das Metall der Gruppe VIII Nickel ist und die ungesättigte organische Verbindung 1,3-Butadien ist.

**17.** Hydrocyanierungsverfahren nach Anspruch 16, wobei das HCN weniger als 20 ppm Schwefeldioxid, weniger als 40 ppm Schwefelsäure, weniger als 20 ppm Cyanogen, weniger als 10 ppm Epoxid, weniger als 20 ppm Acrylnitril und weniger als 100 ppm Peroxide enthält und das 1,3-Butadien weniger als 5 ppm t-Butylcatechol, weniger als 500 ppm Vinylcyclohexen und weniger als 100 ppm Peroxide enthält.

**18.** Hydroformylierungsverfahren, umfassend Inkontaktbringen einer ungesättigten organischen Verbindung mit CO und $H_2$ in Anwesenheit einer Katalysatorzusammensetzung, umfassend mindestens eine Zusammensetzung nach Anspruch 2 und mindestens ein Metall der Gruppe VIII.

**19.** Hydroformylierungsverfahren nach Anspruch 18, wobei das Metall der Gruppe VIII Rhodium, Iridium oder Platin ist.

**20.** Hydroformylierungsverfahren nach Anspruch 19, wobei die ungesättigte organische Verbindung aus der Gruppe ausgewählt ist, bestehend aus 3-Pentennitril, 3-Pentensäure, 3-Pentenal, Allylalkohol und Alkyl-3-pentenoat oder einem Gemisch davon.

**21.** Hydroformylierungsverfahren nach Anspruch 20, wobei die ungesättigte organische Verbindung weniger als 100 ppm Peroxide enthält und das Metall der Gruppe VIII Rhodium ist.

**22.** Isomerisierungsverfahren, umfassend Umsetzen einer ungesättigten organischen Nitrilverbindung in Anwesenheit einer Katalysatorzusammensetzung, umfassend mindestens eine Zusammensetzung nach Anspruch 2 und mindestens ein Metall der Gruppe VIII.

**23.** Isomerisierungsverfahren nach Anspruch 22, wobei das Metall der Gruppe VIII Nickel, Palladium oder Cobalt ist.

**24.** Isomerisierungsverfahren nach Anspruch 23, wobei die ungesättigte organische Nitrilverbindung 2-Methyl-3-butennitril ist und das Metall der Gruppe VIII Nickel ist.

**25.** Isomerisierungsverfahren nach Anspruch 24, wobei das 2-Methyl-3-butennitril weniger als 100 ppm Peroxide enthält.

## Revendications

**1.** Processus de préparation d'une composition polymère contenant du phosphore par :

(1) le couplage oxydatif aryle-aryle d'un monomère répondant à la structure :

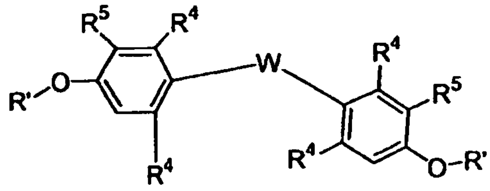

où :

W est sélectionné parmi le groupe consistant en arylène en $C_6$-$C_{20}$, alkylène en $C_{1}$-$C_{20}$ et cycloalkylène ; chaque R' représente individuellement hydrogène ou un groupement protecteur hydroxyle sélectionné parmi le groupe constitué par, sans être limité par, alkyle, alkoxyalkyle, un carbonylalkyle ou un éther-couronne formé en prenant les deux radicaux R' ensembles ;
chaque $R^4$ représente indépendamment H, alkyle en $C_1$-$C_{20}$ à chaîne ramifiée ou linéaire, cycloalkyle en $C_1$-$C_{20}$ ou aryle en $C_6$-$C_{20}$ ;
chaque $R^5$ représente indépendamment alkyle en $C_1$-$C_{20}$ à chaîne ramifiée ou linéaire, cycloalkyle en $C_1$-$C_{20}$ ou aryle en $C_6$-$C_{20}$ ; et

(2) la conversion de R' par un substituant sélectionné parmi -P(-O-Ar)$_2$, - P(Ar)$_2$ et -P(Ar)(-O-Ar), où chaque Ar représente individuellement phényle, phényle substitué, naphtyle ou naphtyle substitué, à condition que les deux groupements Ar qui sont directement ou indirectement liés au même atome de phosphore puissent être liés l'un à l'autre par une unité de liaison sélectionnée parmi les suivantes : liaison directe, alkylidène, amine secondaire ou tertiaire, oxygène, sulfure, sulfone et sulfoxyde; et chaque Ar pouvant de plus être substitué par alkyle en $C_1$-$C_{20}$ à chaîne ramifiée ou linéaire, cycloalkyle en $C_1$-$C_{20}$, aryle en $C_6$-$C_{20}$, acétal, cétal, cycloalkoxy, aryloxy, perhaloalkyle, fluor, chlore, brome, formyle, ester, hydrocarbylsulfinyle, hydrocarbylsulfonyle, hydrocarbylcarbonyle, éther cyclique, $-OR^3$, $-CO_2R^3$, $-SO_3R^3$, - $S(O)R^3$, $-SO_2R^3$, -CHO, $-C(O)R^3$ et CN, où chaque $R^3$ représente indépendamment alkyle en $C_1$-$C_{20}$ à chaîne ramifiée ou linéaire, cycloalkyle en $C_1$-$C_{20}$ ou aryle en $C_6$-$C_{20}$.

**2.** Composition polymère contenant du phosphore qui peut être obtenue par (1) le couplage oxydatif aryle-aryle d'un monomère, ledit monomère répondant à la structure :

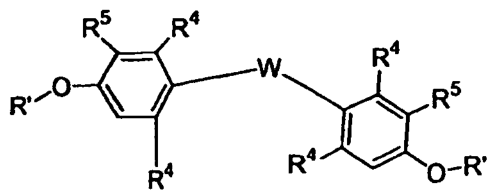

où :

W est sélectionné parmi le groupe consistant en arylène en $C_6$-$C_{20}$, alkylène en $C_{1}$-$C_{20}$ et cycloalkylène ;
chaque R' représente individuellement hydrogène ou un groupement protecteur hydroxyle sélectionné parmi le groupe constitué par, sans être limité par, alkyle, alkoxyalkyle, carbonylalkyle ou un éther-couronne formé par les deux radicaux R' ;
chaque $R^4$ représente indépendamment H, alkyle en $C_1$-$C_{20}$ à chaîne ramifiée ou linéaire, cycloalkyle en $C_1$-$C_{20}$ ou aryle en $C_6$-$C_{20}$ ;
chaque $R^5$ représente indépendamment alkyle en $C_1$-$C_{20}$ à chaîne ramifiée ou linéaire, cycloalkyle en $C_1$-$C_{20}$ ou aryle en $C_6$-$C_{20}$ ; et

(2) par la conversion de R' par un substituant sélectionné parmi -P(-O-Ar)$_2$, - P(Ar)$_2$ et -P(Ar)(-O-Ar), où chaque Ar représente individuellement phényle, phényle substitué, naphtyle ou naphtyle substitué, à condition que les deux groupements Ar qui sont directement ou indirectement liés au même atome de phosphore puissent être liés l'un à l'autre par une unité de liaison sélectionnée parmi les suivantes : liaison directe, alkylidène, amine secondaire ou tertiaire, oxygène, sulfure, sulfone et sulfoxyde; et chaque Ar pouvant de plus être substitué par alkyle en $C_1$-$C_{20}$

à chaîne ramifiée ou linéaire, cycloalkyle en $C_1$-$C_{20}$, aryle en $C_6$-$C_{20}$, acétal, cétal, cycloalkoxy, aryloxy, perhaloalkyle, fluor, chlore, brome, formyle, ester, hydrocarbylsulfinyle, hydrocarbylsulfonyle, hydrocarbylcarbonyle, éther cyclique, $-OR^3$, $-CO_2R^3$, $-SO_3R^3$, - $S(O)R^3$, $-SO_2R^3$, -CHO, $-C(O)R^3$ et CN, où chaque $R^3$ représente indépendamment alkyle en $C_1$-$C_{20}$ à chaîne ramifiée ou linéaire, cycloalkyle en $C_1$-$C_{20}$ ou aryle en $C_6$-$C_{20}$.

**3.** Composition catalytique comprenant au moins une composition polymère contenant du phosphore selon la revendication 2 et au moins un métal du Groupe VIII.

**4.** Composition catalytique selon la revendication 3, où ledit métal du Groupe VIII est nickel, palladium, cobalt.

**5.** Composition catalytique selon la revendication 3, où ledit métal du Groupe VIII est rhodium, iridium et platine.

**6.** Composition catalytique selon la revendication 4, où ledit métal du Groupe VIII est le nickel.

**7.** Composition catalytique selon la revendication 4, qui comprend également un acide de Lewis.

**8.** Composition catalytique selon la revendication 7, où ledit acide de Lewis est sélectionné parmi le groupe consistant en $ZnBr_2$, $ZnI_2$, $ZnCl_2$, $ZnSO_4$, $CuCl_2$, CuCl, $Cu(O_3SCF_3)_2$, $CoCl_2$, $CoI_2$, $FeI_2$, $FeCl_3$, $FeCl_2$, $FeCl_2(THF)_2$, $TiCl_4(THF)_2$, $TiCl_2$, $ClTi(OiPr)_2$, $MnCl_2$, $ScCl_3$, $AlCl_3$, $(C_8H_{17})AlCl_2$, $(C_8H_{17})_2AlCl$, $(iso\text{-}C_4H_9)_2AlCl$, $Ph_2AlCl$, $PhAlCl_2$, $ReCl_5$, $ZrCl_4$, $NbCl_5$, $VCl_3$, $CrCl_2$, $MoCl_5$, $YCl_3$, $CdCl_2$, $LaCl_3$, $Er(O_3SCF_3)_3$, $Yb(O_2CCF_3)_3$, $SmCl_3$, $B(C_6H_5)_3$, $TaCl_5$ et $(C_6H_5)SnX$, où X peut représenter $CF_3SO_3$, $CH_3C_6H_5SO_3$ ou $(C_6H_5)_3BCN$.

**9.** Composition catalytique selon la revendication 8, où ledit acide de Lewis est chlorure de zinc ou chlorure de fer.

**10.** Processus d'hydrocyanation qui comprend la mise en contact d'un composé organique insaturé avec de l'HCN en présence d'une composition catalytique comprenant au moins une composition selon la revendication 2 et au moins un métal du Groupe VIII ainsi qu'un acide de Lewis en option.

**11.** Processus d'hydrocyanation selon la revendication 10, où ledit acide de Lewis est sélectionné parmi le groupe consistant en $ZnBr_2$, $ZnI_2$, $ZnCl_2$, $ZnSO_4$, $CuCl_2$, CuCl, $Cu(O_3SCF_3)_2$, $CoCl_2$, $CoI_2$, $FeI_2$, $FeCl_3$, $FeCl_2$, $FeCl_2(THF)_2$, $TiCl_4(THF)_2$, $TiCl_2$, $ClTi(OiPr)_2$, $MnCl_2$, $ScCl_3$, $AlCl_3$, $(C_8H_{17})AlCl_2$, $(C_8H_{17})_2AlCl$, $(iso\text{-}C_4H_9)_2AlCl$, $Ph_2AlCl$, $PhAlCl_2$, $ReCl_5$, $ZrCl_4$, $NbCl_5$, $VCl_3$, $CrCl_2$, $MoCl_5$, $YCl_3$, $CdCl_2$, $LaCl_3$, $Er(O_3SCF_3)_3$, $Yb(O_2CCF_3)_3$, $SmCl_3$, $B(C_6H_s)_3$, $TaCl_5$ et $(C_6H_5)SnX$, où X peut représenter $CF_3SO_3$, $CH_3C_6H_5SO_3$ ou $(C_6H_5)_3BCN$.

**12.** Processus d'hydrocyanation selon la revendication 11, où ledit acide de Lewis est chlorure de zinc ou chlorure de fer.

**13.** Processus d'hydrocyanation selon la revendication 12, où ledit métal du Groupe VIII est nickel, palladium ou cobalt.

**14.** Processus d'hydrocyanation selon la revendication 13, où ledit composé organique insaturé est le 3-pentènenitrile ou le 4-pentènenitrile et ledit métal du Groupe VIII est le nickel.

**15.** Processus d'hydrocyanation selon la revendication 14, où le HCN contient moins de 20 ppm de dioxyde de soufre, moins de 40 ppm d'acide sulfurique, moins de 20 ppm de cyanogène, moins de 10 ppm d'époxyde, moins de 20 ppm d'acrylonitrile et moins de 100 ppm de peroxydes et lesdits pentènenitriles contiennent moins de 100 ppm de peroxydes.

**16.** Processus d'hydrocyanation selon la revendication 15, où ledit métal du Groupe VIII est le nickel et ledit composé organique insaturé est le 1,3-butadiène.

**17.** Processus d'hydrocyanation selon la revendication 16, où le HCN contient moins de 20 ppm de dioxyde de soufre, moins de 40 ppm d'acide sulfurique, moins de 20 ppm de cyanogène, moins de 10 ppm d'époxyde, moins de 20 ppm d'acrylonitrile et moins de 100 ppm de peroxydes et ledit 1,3-butadiène contient moins de 5 ppm de catéchol de t-butyle, moins de 500 ppm de cyclohexène de vinyle et moins de 100 ppm de peroxydes.

**18.** Processus d'hydroformylation qui comprend la mise en contact d'un composé organique insaturé avec du CO et de l'$H_2$ en présence d'une composition catalytique comprenant au moins une composition selon la revendication 2 et au moins un métal du Groupe VIII.

**19.** Processus d'hydroformylation selon la revendication 18, où ledit métal du Groupe VIII est rhodium, iridium ou platine.

**20.** Processus d'hydroformylation selon la revendication 19, où ledit composé organique insaturé est sélectionné parmi le groupe consistant en 3-pentènenitrile, acide 3-pentènoïque, 3-pentènal, alcool allylique, 3-pentènoate d'alkyle ou un mélange de ceux-ci.

**21.** Processus d'hydroformylation selon la revendication 20, où ledit composé organique insaturé contient moins de 100 ppm de peroxydes et ledit métal du Groupe VIII est le rhodium.

**22.** Processus d'isomérisation qui comprend la réaction d'un composé organique insaturé à fonction nitrile en présence d'une composition catalytique comprenant au moins une composition selon la revendication 2 et au moins un métal du Groupe VIII.

**23.** Processus d'isomérisation selon la revendication 22, où ledit métal du Groupe VIII est nickel, palladium ou cobalt.

**24.** Processus d'isomérisation selon la revendication 23, où ledit composé organique insaturé à fonction nitrile est le 2-méthyl-3-butènenitrile et ledit métal du Groupe VIII est le nickel.

**25.** Processus d'isomérisation selon la revendication 24, où le 2-méthyl-3-butènenitrile contient moins de 100 ppm de peroxydes.